# EUROPEAN PATENT SPECIFICATION

(11) **EP 2 010 039 B1**
(45) Date of publication and mention of the grant of the patent: **03.09.2014**
(21) Application number: 06846606.9
(22) Date of filing: 13.12.2006
(51) Int. Cl.: A61B 1/00

(54) **FLEXIBLE ENDOSCOPE WITH VARIABLE STIFFNESS SHAFT**
FLEXIBLES ENDOSKOP MIT SCHAFT VARIABLER STEIFHEIT
ENDOSCOPE FLEXIBLE AVEC TIGE A RIGIDITE VARIABLE

(30) Priority: 31.03.2006 US 396350
(43) Date of publication of application: 07.01.2009
(73) Proprietor: Boston Scientific Limited, Christ Church (BB)
(72) Inventor: MACNAMARA, Francis, T., Newton, MA 02466 (US); DAYTON, Peter, L., Brookline, MA 02445 (US); CASTRO, Ian, Woburn, MA 01801 (US); MASEDA, Luis, J., Natick, MA 01760 (US); SBARDELLI, John, Sturbridge, MA 01566 (US); ZOCCHI, Michael, R., Somerville, MA 02144 (US); HARRIS, Edward, M., Pompton Plains, NJ 07444 (US); BOULAIS, Dennis, R., Danielson, CT 06239 (US); BANIK, Michael, S., Boston, MA 01740 (US); CHIN, Albert, C C., Newton, MA 02461 (US)
(74) Representative: Potter Clarkson LLP
(86) International application number: PCT/US2006/062041
(87) International publication number: WO 2007/114869

(56) References cited:
- EP-A- 1 123 714
- EP-A1- 0 835 637
- EP-A1- 0 916 359
- WO-A-93/23111
- GB-A- 191 303 531
- US-A1- 4 669 172
- US-A1- 4 719 924

## Description

### FIELD OF THE INVENTION

The present invention relates to medical devices in general, and to endoscopes in particular.

### BACKGROUND OF THE INVENTION

As an alternative to performing more invasive medical procedures, many physicians are utilizing endoscopes and catheters to perform diagnostic and therapeutic procedures on internal tissues of patients. With this less invasive approach, a medical device such as an endoscope or catheter is advanced to a site of interest and the procedure is performed. Most endoscopes and some catheters comprise a steerable shaft that is advanced through the patient's anatomy until the distal tip reaches the point of interest.

To withstand the forces required to advance the endoscope through the anatomy while allowing the device to be steered, some endoscopes and catheters have a variable stiffness along their shafts. The tip of the device is generally made flexible to allow it to be steered, while the proximal end is stiffer to transmit the forces applied by a physician during use.

In reusable endoscopes, the spiral is not modified, and such variable flexibility is typically provided by varying the material from which an outer sheath of the shaft is made or by varying the characteristics of a braid within the sheath. While both approaches work, shafts made in this way can be expensive to manufacture.

WO 93/23111 describes a steerable endoscope that has a section of enhanced bending characteristics. An outer spring of the endoscope has widely spaced convolutions in the bending section and narrowly spaced or closed convolutions on either side of the bending section. Where the convolutions are widely spaced, the bending of the spring is greatly enhanced.

EP 1 123 714 A1 discloses a catheter having a shaft with a coil wound at different winding pitches.

If a device, such as an endoscope or catheter, is intended for single-use application, the device must be inexpensive enough to manufacture such that it can be used on a single patient and then thrown away. The shaft of a single-use endoscope should have performance characteristics that are equal to or better than those of a conventional endoscope while being less expensive to manufacture.

### SUMMARY OF THE INVENTION

As will be described in further detail below, the present invention is a shaft for use in a medical device, such as an endoscope or catheter, having a flexibility that is variable along its length. The shaft of the invention is defined in claim 1.

### BRIEF DESCRIPTION OF THE DRAWINGS

The foregoing aspects and many of the attendant advantages of this invention will become more readily appreciated as the same become better understood by reference to the following detailed description, when taken in conjunction with the accompanying drawings, wherein:
FIGURE 1 illustrates one embodiment of a shaft for use in a medical device in accordance with an embodiment of the present invention;
FIGURE 2 is a cross-sectional illustration of a spiral wrap and sheath having a variable flexibility in accordance with an embodiment of the present invention;
FIGURE 3 illustrates a spiral wrap in a shaft having a varying flexibility in accordance with an embodiment of the present invention;
FIGURE 4 illustrates a spiral wrap in a shaft having a varying flexibility in accordance with yet another embodiment of the present invention;
FIGURE 5 illustrates a spiral wrap in a shaft having a varying flexibility in accordance with yet another embodiment of the present invention;
FIGURE 6 illustrates a spiral wrap in a shaft having a varying flexibility in accordance with yet another embodiment of the present invention;
FIGURE 7 illustrates one technique for fixing a spiral wrap within a shaft of a medical device in accordance with an embodiment of the present invention;
FIGURE 8 illustrates another technique for fixing a spiral wrap within a shaft of a medical device for use in tracking the anatomy in accordance with an embodiment of the present invention;
FIGURE 9 is an illustration of an endoscope having a variable flexibility shaft in accordance with an embodiment of the present invention; and
FIGURE 10 illustrates a slotted tube that can replace removed sections of a spiral wrap in accordance with yet another embodiment of the present invention.

### DETAILED DESCRIPTION OF THE PREFERRED EMBODIMENT

As indicated above, embodiments in accordance with the present invention are related to methods and shafts for use in a medical device having a flexibility that can vary along its length. In the embodiments disclosed, the shaft is used in an endoscope. However, it will be appreciated that the present invention can also be used in catheters or other minimally invasive devices. In a co-pending application titled "Flexible Device Shaft with Angled Spiral Wrap," Attorney Docket No. BSEN126408, applicant describes a flexible spiral wrap having wraps with angled, multi-planar, arcuate and irregular surface edges, which also are used to adjust shaft flexibility. This application is incorporated herein expressly by reference.

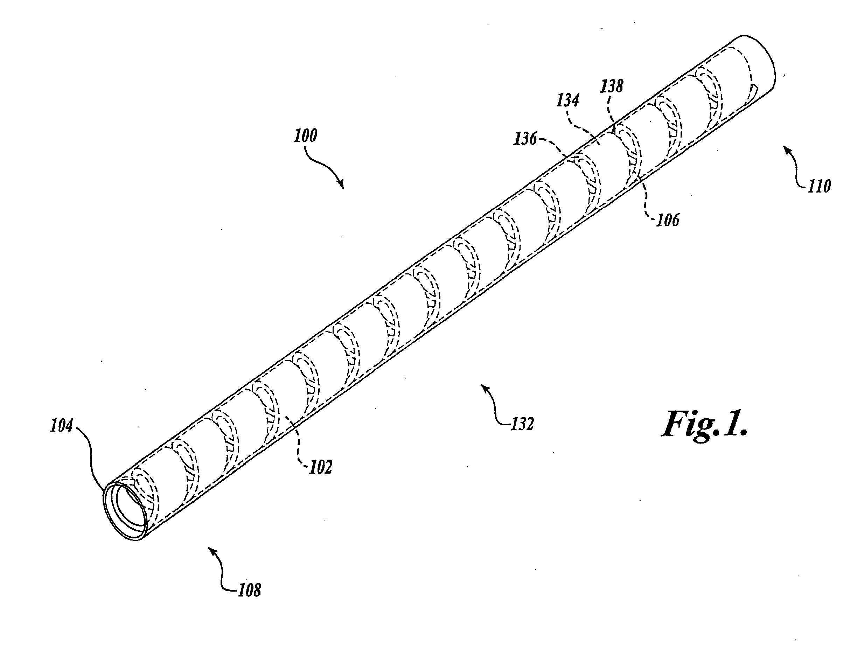

FIGURE 1 is an illustration of a portion of a shaft 100 having a flexibility that varies along the length of the shaft. In one embodiment, the shaft is constructed as a spiral wrap 102 that is overlaid with a sheath including a polyurethane wrapper (not shown) to cover the spiral wrap. A braid (also not shown) is placed over the wrapper to provide torsional strength to the shaft. Finally, the braid is covered with a polyurethane coating by extrusion, dipping, or like processes. It is to be appreciated that the shafts may include other devices, such as a complementary metal-oxide semi-conductor (CMOS) for an image sensor and light emitting diodes (LEDs) for illumination, and one or more control wires for steering, etc. These additional components are not shown or discussed in this application for brevity. Furthermore, the sheath 104 that is illustrated has a single layer for clarity. However, it is to be appreciated that the sheath 104 is merely representative and may have more than one layer. Additionally, the various embodiments of the shafts can be used in a single-use endoscope. One example of a single-use endoscope is described in U.S. Patent Application Publication No. 20050197536, dated September 8, 2005.

The spiral wrap 102 is a helical coil including a number of individual wraps, such as wrap 134. Each individual wrap may be defined by starting at any position on the spiral wrap 102, continuing along the spiral 102 until tracking one complete 360° revolution. Each individual wrap has a leading surface edge 136 and a trailing surface edge 138. The adjacent wraps are separated from one another by a gap 106. The "gap spacing" is the distance between the leading surface edge of any wrap and the trailing surface edge of an adjacent wrap. Generally, without modification, the gap spacing 106 between wraps in the spiral wrap 102 is uniform along the length of the spiral wrap 102. In addition, the individual wraps of the spiral wrap 102 are uniform in both width and thickness. A spiral wrap 102 that has uniform wraps 134 and uniform gaps 106 generally has a substantially constant flexibility along its length.

As discussed above, embodiments of the present invention include modifying the spiral wrap within the shaft to vary its stiffness along the length of the shaft or to change the stiffness along the shaft if it already has a varying stiffness due to other techniques, such as a change in braiding along its length or the use of different materials in the shaft coating, etc. Embodiments of the present invention include one or more of (1) varying the gap spacing between two or more wraps in the spiral wrap, (2) varying the wrap width of one or more wraps, (3) varying the wrap thickness of one or more wraps, and (4) changing the material of construction of the spiral wrap. Any or all of the techniques (1) through (4) above can be performed at any location of the spiral wrap 102 for any number of wraps.

In one embodiment of the present invention, the flexibility of a section of a spiral wrap, such as spiral wrap 102, can be increased by increasing the gap spacing along a portion or whole of one or all of the distal section 108, the center section 132, and the proximal section 110.

The gap spacing 106 between the adjacent wraps, such as wrap 134, of the spiral wrap 102 is generally small. In one embodiment, the shaft 100 is used for the center section 132 and proximal section 110 of the endoscope's shaft without modification. The small gap spacing 106 gives the shaft 100 adequate column strength in the center 132 and proximal 110 sections. However, the gap spacing 106 in the spiral wrap 102 is increased in the distal section 108 to provide additional flexibility where such flexibility is desired. Alternatively, embodiments of the present invention include a shaft having a spiral wrap using the original gap spacing at the distal section 108 and reducing the gap spacing at the center section 132 and/or the proximal section 110. The gap spacing 106 of a spiral wrap 102 may be decreased, or increased, or any combination of increasing or decreasing the gap spacing 106 in any one or all of the distal section 108, center section 132, and proximal section 110. The following description of a representative method to increase the gap spacing 106 in the distal section serves to illustrate one exemplary method that can be applied to increase or decrease the gap spacing 106 of one or more section.

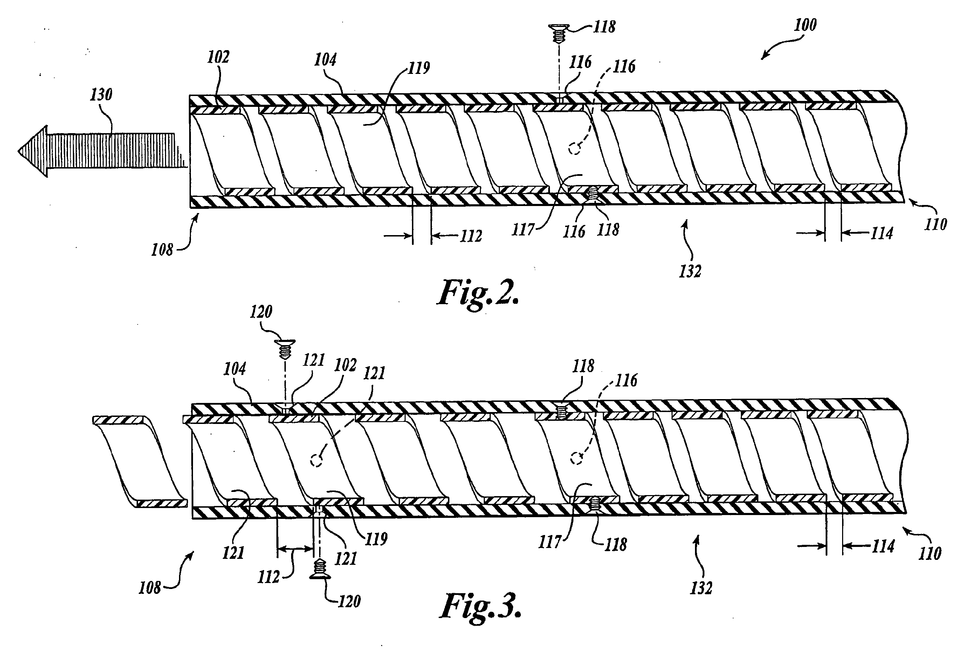

FIGURE 2 is an illustration of a cross section of a modified shaft 100 having a spiral wrap 102 and cover sheath 104 in accordance with one embodiment of the present invention. As discussed above, the spiral wrap 102 has a distal section 108, a proximal section 110, and a center section 132, wherein all three sections initially have about the same stiffness or flexibility. As a representative example of changing flexibility by changing gap spacing, the flexibility in the distal section 108 will be increased. In this example, the proximal section 110 and the center section 132 of the spiral wrap 102 are assumed not to require modification. The unmodified spiral wrap 102 has gap spacing 112 in the distal section 108 that is similar to the gap spacing 114 in the proximal section 110 and in the center section 132.

In an embodiment of the present invention, the cover sheath 104 is secured, or anchored, to the spiral wrap 102 other than merely by the friction caused by the spiral wrap 102 pressing against the cover sheath 104. One method for achieving this added attachment is to use mechanical fasteners, such as metal screws, pins, rivets, etc. Alternative attachment means include plastic pins, or rivets or welds, which can be ultrasonically created or glued in place and/or reshaped by heat and/or pressure. Staples (metal, plastic, or other materials) may also be used to secure the spiral wrap 102 by driving the staples through the cover sheath 104 and spiral wrap 102 and be reshaped with an anvil in the inner diameter (or, inversely, the outer diameter) of the shaft 100. A stamped ring with bent tangs may be used and swaged into the outer diameter of the cover sheath 104 and into the inner spiral wrap 102. Alternatively, adhesives may be used to adhere the spiral wrap 102 to the inner surface of cover sheath 104. The spiral wrap 102 may be heat staked to the inner surface of the cover sheath 104. In this example, three countersunk holes 116 are drilled and tapped through the cover sheath 104 that extend into, but do not penetrate, the spiral wrap 117. In one embodiment, the holes 116 are about 6 to 12 inches from the end of the distal section 108 of the shaft 102, preferably 8 to 10 inches. The countersunk holes 116 are placed at 120° from each other around the perimeter of the cover sheath 104. Screws 118 are threaded into countersunk holes 116 at the three locations. Alternatively, additional or fewer countersunk holes 116 and screws 118 may be used. Once the screws 118 are threaded into countersunk holes 116, it can be appreciated that the cover sheath 104 and spiral wrap 102 are stationary with respect to each other at the wrap 117. However, the spiral wrap 102 can slide relative to the cover sheath 104 in the distal section 108. With the spiral wrap 102 affixed to the cover sheath 104 at the location of wrap 117, the distal section 108 of the spiral wrap 102 is pulled in the direction indicated by the arrow 130 to increase the gap spacing.

The spiral 102 can be pulled out directly or it can be unwound as it is pulled out. The spiral 102 will naturally reduce in diameter as it is expanded in length. By unwinding the spiral 102 as it is withdrawn, the diameter can be maintained. This ensures a continued close fit between spiral 102 and sheath 104 and prevents any risk of kinking or collapsing of the outer sheath 104. If the spiral 102 is pulled without unwinding, then the slight reduction in diameter can help to further reduce the bending stiffness in this region.

As illustrated in FIGURE 3, the pulling force will cause the gap spacing 112 in the distal section 108 forward of the wrap 117 to increase. About one (1) inch of additional gap spacing 112 is added in the distal section 108. With the gap spacing 112 in the increased configuration, three additional countersunk holes 121 are provided in cover sheath 104 and spiral wrap 102 in the distal section 108 at wrap 119. Screws 120 can be placed into drilled and tapped countersunk holes 121 in the wrap 119 to hold the spiral wrap 102 in the second post-stressed configuration. Any spiral wrap 102 material that is in excess and is beyond the end of the cover sheath 104 can be trimmed and discarded. A spiral wrap 102 having greater gap spacing 112 in the distal section 108 as compared to the gap spacing 114 in either the proximal section 110 or the center section 132 is produced. The additional gap spacing between wraps in the distal section 108 provides the spiral wrap 102 with greater flexibility in tight bends with a small loss of column strength. In general, wraps with the largest gap spacing will provide the greatest flexibility in the spiral, other things being equal.

In other embodiments of the present invention, the flexibility or stiffness can be changed at several locations on a shaft. The shaft may be manufactured with very tightly packed wraps in the proximal section. The spiral could then be anchored and spread by a small amount in the center section of the shaft, and then anchored again. The spiral would then be spread by a larger amount in the distal section to obtain the greatest flexibility at the distal section. The center section can have a transitional flexibility between the high column strength of the proximal section and the highly flexible distal section. This prevents any sharp transitions in the shaft flexibility and may increase the ability of the shaft to track correctly through a patient's anatomy. Different sections of the shaft can be isolated, which enables different sections of the shaft to have different properties.

A further advantage of an embodiment of the present invention is that the spiral wrap can be manufactured on a continuous basis with any change to the gap spacing being performed as a secondary operation. This is a much less expensive method for varying the shaft's stiffness when compared to varying the durometer of the shaft cover, varying the braid angle, or manufacturing the shaft in cut lengths. With plastic, the spiral advantageously does not have to be cut to change the gap spacing. The spiral remains as one unitary piece in the shaft so the risk of kinking is minimized. The shaft manufacturing process can be run at appropriate conditions to give the required column strength to the center and proximal sections of the shaft. Afterwards, the spiral is secured to the cover sheath to maintain the performance in these sections while the distal section is adjusted to give the required flexibility.

In other embodiments of the present invention, the flexibility of the spiral is changed by varying the wrap width and/or the wrap thickness of one or more wraps at any location along the spiral. In some embodiments of the present invention, the spiral may need to be cut. For example, to reduce the stiffness (increase the flexibility), a distal section of the spiral wrap can be cut. The remaining less flexible wrap can be used in the proximal section and a more flexible spiral wrap can be used in the distal section. This can be accomplished either with a distal section spiral having wraps with thinner walls, a reduced width, or a spiral made from a more flexible material. The result is a shaft with higher stiffness in the center and proximal sections of the shaft and higher flexibility in the distal section. Varying the wrap thickness and/or wrap width and/or wrap material provides inexpensive methods to produce a shaft having variable stiffness.

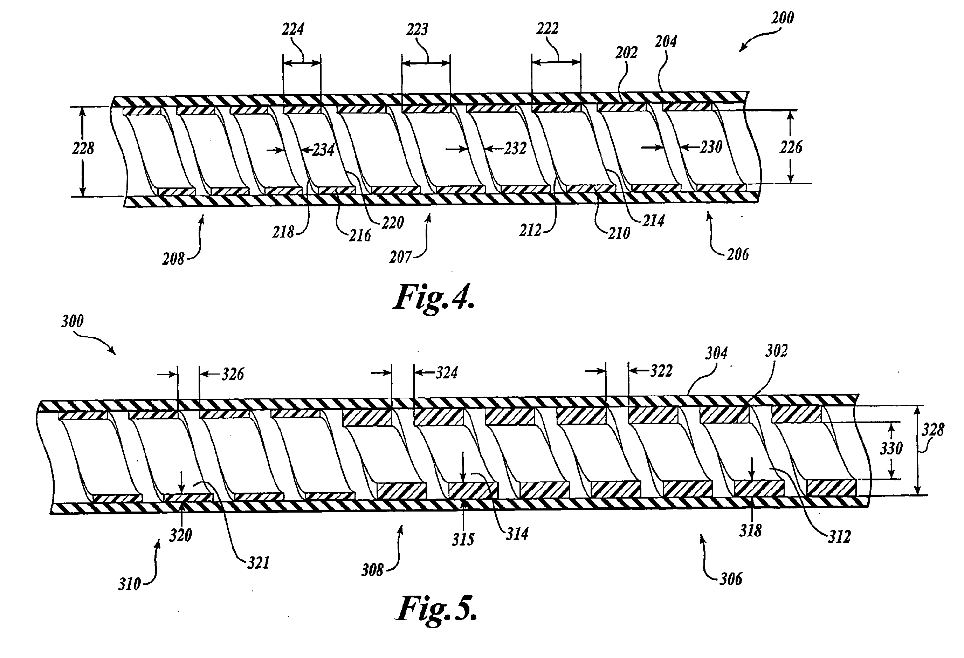

Referring to FIGURE 4, a shaft 200 having a spiral wrap 202 and cover sheath 204 is illustrated. The shaft 200 has a proximal section 206, a distal section 208, and a center section 207 between the proximal section 206 and the distal section 208. Wraps, such as wrap 210 in the proximal section 206, have a wrap width 222, which is defined as the distance between a leading surface edge 212 and a trailing surface edge 214. In one embodiment, the wrap width 222 of wraps in the proximal section 206 can vary between 3/8" (inches) and 1/2" (inches). Wraps, such as wrap 216 in the distal section 208, have a relatively smaller wrap width 224 from the leading surface edge 218 to the trailing surface edge 220, as compared to the wrap width 222 in the proximal section 206. In one example, the wrap width 224 of wraps in the distal section 208 can vary between 1/4" (inches) and 3/8" (inches). The wrap width 223 of wraps in the center section 207 can be larger than the wrap width 224 in the distal section 208, and the same or smaller than the wrap width 222 in the proximal section 206. The gap spacing 230 in the proximal section 206 can be the same or different from the gap spacing 234 in the distal section 208 and/or the gap spacing 232 in the center section 207. Alternatively, the gap spacing 234 in the distal section 208, the gap spacing 232 in the center section 207, and the gap spacing 230 in the proximal section 206 can be different from one another. A spiral wrap 202 having a wrap width 224 that is small in the distal section 108, as compared to the wrap width 230 in the proximal section 206 and the wrap width 223 in the center section 207, has greater flexibility in the distal section 208. Generally, wraps with the smallest wrap width will provide the greatest flexibility in the spiral, other things being equal.

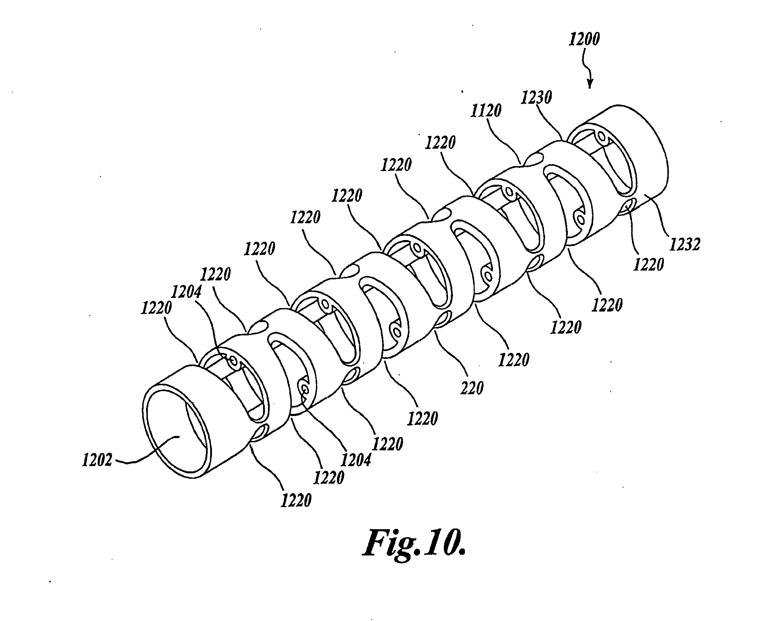

One embodiment in accordance with the present invention is a method for providing a smaller wrap width 224 in the distal section 208. The method includes obtaining a spiral wrap, such as spiral wrap 102 (FIGURE 1), and cutting a section from the distal section 108 from the pre-modified spiral wrap 102 before or after insertion into the cover sheath 104. A different spiral wrap having a smaller wrap width can be used to replace the removed section. In this way, the stiffness of the distal section 208 can be varied as compared to the center section 207 and the proximal section 206. The new distal section 208 can be welded via ultrasound, laser, or the like to the center section 207. The cover sheath 204 is made from an ultraviolet (UV) transparent material that allows laser welding through the cover sheath 204 of the distal section 208 to the center section 207. Alternatively, adhesives or mechanical fasteners can be used to attach the newer distal section 208 to the spiral wrap 202. Alternatively, any section of the spiral wrap can be removed and replaced with a spiral wrap section that has a greater, a smaller, or the same wrap width than the section that was removed. Changing the flexibility by replacing the removed section with a spiral wrap having the same wrap width generally includes using a different material for the new section. Further still, the flexibility of the spiral wrap can be changed by replacing the removed spiral wrap section with a different design, such as the slotted tube illustrated in FIGURE 10. The slotted tube may have a thinner wall and more flexibility than the unmodified spiral wrap. The slotted tube has the advantage of ensuring adequate column strength while reducing flexibility. The methods described earlier for reducing stiffness at the distal end would also reduce the column strength.

An alternate technique for achieving a varying wrap width 224 in the spiral wrap includes adjusting a cutting instrument, which cuts the wall of a plastic tube as the tube is being extruded. Such extrusion process carries the tube forward along an axial direction. A cutting instrument is rotated around the tube as it is extruded to the wall of the tube while the tube is moving forward. If the speed of the extrusion and the rotational speed of the cutting instrument are kept constant, the spiral wrap has wraps of uniform width. However, either the speed of the cutting instrument or the extrusion rate can be varied to change the width of the wraps.

One embodiment in accordance with the present invention is a tube having a distal section with smaller wrap thickness as compared to the proximal section. Referring to FIGURE 5, a shaft 300 having a spiral wrap 302 and cover sheath 304 is illustrated. The shaft 300 has a proximal section 306, a distal section 310, and a center section 308 disposed between the proximal section 306 and the distal section 310. The spiral wrap 302 includes a series of wraps disposed from the proximal section 306 to the distal section 310. The gap spacing 322 in the proximal section 306 can be the same or different from the gap spacing 324 in the center section 308 or from the gap spacing 326 in the distal section 310. Each wrap in the distal section 310, the center section 308, and the proximal section 306 has a thickness, which is measured between the outer diameter 328 and the inner diameter 330 of the spiral wrap 302. This dimension is referred to as the wrap thickness in this application. In one embodiment, the outer diameter of the spiral wrap 302 can range from 7.5 mm to 11.5 mm and the inner diameter of the spiral wrap 302 can range from 6.5 mm to 11.0 mm. Wraps, such as wrap 312, in the proximal section 306 have a wrap thickness 318 that can be the same or different compared to the wrap thickness 315 of wraps, such as wrap 314, in the center section 308. Wraps, such as wrap 321, in the distal section 310 have a wrap thickness 320 that is less than the wrap thickness 318 in the proximal section 306, and less than the wrap thickness 315 in the center section 308. Generally, wraps with the smallest wrap thickness will provide the greatest flexibility in the spiral, other things being equal. Thinner wall spirals have the added advantage of creating more free space on the inside of the shaft. This reduces the wall-to-wall friction between the shaft inner diameter (ID) and the tube bundle. This helps to further reduce the bending stiffness of the distal end of the shaft.

One embodiment in accordance with the present invention is a method for providing a smaller wrap thickness 320 in the distal section 310. The method includes cutting a portion from the distal section 310 from a pre-modified spiral before or after assembling the shaft. A different spiral wrap having a thinner wrap thickness 320 can be secured to the distal section 310 to complete the spiral wrap. In this way, the stiffness of the distal section 310 can be varied as compared to the center section 308 and the proximal section 306. The new distal section 310 can be welded to the center section 308. The cover sheath 304 is made from a UV transparent material that allows welding through the cover sheath 304 of the distal section 310 to the center section 308. Alternatively, adhesives or mechanical fasteners or the like can be used to attach the newer distal section 310 to the spiral wrap 302. Alternatively, any section of the spiral can be removed and replaced with a spiral section that has a greater, a smaller, or the same wrap thickness as the section that was removed. Changing the flexibility by replacing the removed section with a spiral having the same wrap thickness generally includes using a different material for the new section. The slotted tube of FIGURE 10 may be used to replace the removed spiral wrap section to give more flexibility in the distal section.

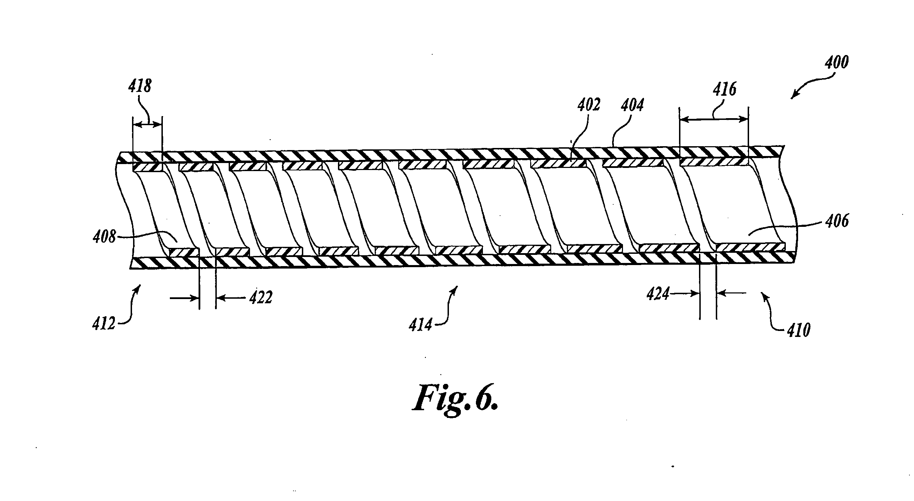

Referring to FIGURE 6, a shaft 400 having a spiral wrap 402 and a cover sheath 404 is illustrated. The shaft 400 has a proximal section 410, a distal section 412, and a center section 414 disposed between the proximal section 410 and the distal section 412. The spiral wrap 402 is a series of wraps from the proximal section 410 to the distal section 412. The gap spacing 424 in the proximal section 410 can be the same or different from the gap spacing 422 in the distal section 412. In this embodiment, the wraps incrementally decrease in wrap width as the wraps approach the distal section 412 from the proximal section 410. For example, the wrap 406 in the proximal section 410 has the largest wrap width 416 in the spiral wrap 402. Wrap 406 can be the proximal end wrap of the spiral wrap 402. The wrap 408 in the distal section 412 has the smallest wrap width 418. Wrap 408 can be the distal end wrap of the spiral wrap 402. The intermediate wraps between wrap 406 and wrap 408 incrementally decrease in wrap width beginning with the wrap that is adjacent to wrap 406 at the proximal section 410, and ending with wrap 408 at the distal section 412. A spiral wrap, such as spiral wrap 402, having wraps of incrementally decreasing wrap width can gradually increase the flexibility of the spiral wrap 402 from the proximal section 410 to the distal section 412 to avoid abrupt changes in flexibility along the spiral wrap 402. Furthermore, incremental changes in flexibility can also apply to incrementally increasing or decreasing the gap spacing and/or the wrap thickness, or all three going from proximal section to distal section.

One embodiment of the present invention is a method for making a spiral having incremental changes in the wrap width. Spiral wrap 402 is made by extruding a plastic tube while a cutting instrument cuts the tube, such that the cutting instrument is accelerated or decelerated along the longitudinal axis of the tube as the tube is being extruded. Alternatively, the velocity of a cutting instrument can be held constant for one wrap, i.e., one complete revolution of the tube, and the velocity is increased (or decreased) during the cutting of the next wrap, i.e., the next complete revolution of the tube.

As can be appreciated from the foregoing discussion, embodiments in accordance with the present invention include a shaft having a spiral wrap, wherein any one or all of the gap spacing, wrap thickness, and wrap width can be increased or decreased for any one or more wraps along the spiral, including incremental changes. Furthermore, combinations of increasing or decreasing any one of the gap spacing, wrap thickness, and wrap width can be implemented for any one or more wraps in one spiral. For example, some spirals can have a different gap spacing applied to two or more wraps, a different wrap width applied to one or more wraps, and a different wrap thickness applied to one or more wraps, all in one shaft. Furthermore, where sections of the spiral wrap are removed and replaced with different spirals or a different tube design, the material used in the newer spiral wrap can be different from the material used in the original spiral wrap or of a different design. For example, where the cover sheath is a polyurethane, the spiral that replaces the removed spiral can also be made from a polyurethane to facilitate either heat staking to the polyurethane surface of the cover sheath or for better adhesive compatibility with the cover. Furthermore, the methods described above and the spirals made therefrom are not limited to plastic spirals. It is possible to change the flexibility of spirals made from metals as discussed above. Changing any one of the gap spacing, wrap width, wrap thickness, material of construction, or tube design advantageously provides the ability to produce a shaft having the desired degree of flexibility or stiffness. Once the desired degree of flexibility or stiffness is achieved through changing the gap spacing, one embodiment in accordance with the present invention is a method to maintain the desired gap spacing along the shaft.

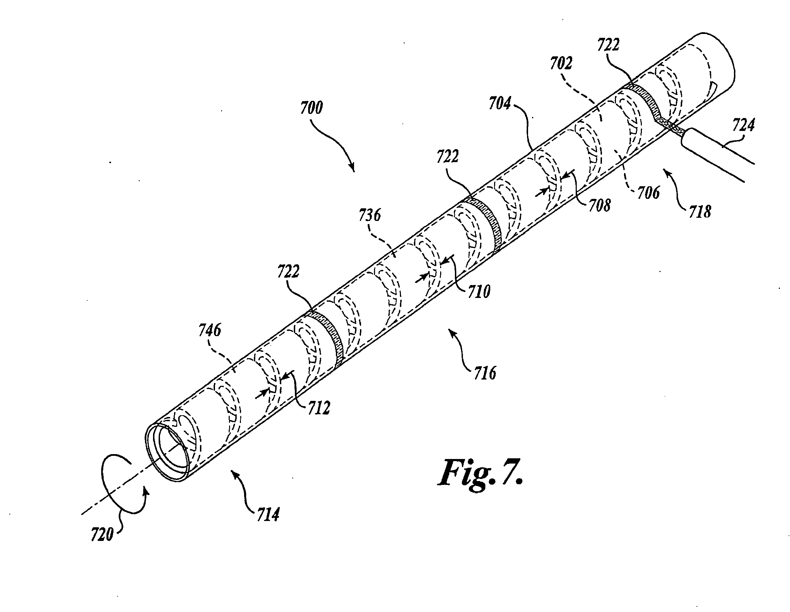

Referring to FIGURE 7, a shaft 700 having a spiral wrap 702 and cover sheath 704 is illustrated. The shaft 700 has a proximal section 718, a distal section 714, and a center section 716 between the proximal section 718 and the distal section 714. Wraps, such as wrap 706 in the proximal section 718, wrap 736 in the center section 716, and wrap 746 in the distal section 714, may or may not have the same or different wrap width, wrap thickness, and gap spacing. The gap spacing 708 in the proximal section 718, or the gap spacing 710 in the center section 716, or the gap spacing 712 in the distal section 714 may or may not have been adjusted as described above. Through normal use of the shaft 700, however, the gap spacing 708 in the proximal section 718, center section 716, and distal section 714 can change throughout the life of the shaft 700. If the gap spacing increases, the increase results in greater flexibility (reduced stiffness) in the affected location. If the gap spacing decreases, the decrease results in reduced flexibility (increased stiffness) in the affected location. For a variety of reasons, it is desirable to maintain the gap spacing. To maintain the gap spacing at the desired setting, the cover sheath 704 is anchored to the spiral wrap 702 at more than one location along the axial direction of the shaft 700. In one embodiment, circumferential welds 722 are provided to weld wraps of the spiral wrap 702 to the cover sheath 704. Circumferential welds 722 may be provided at any location on the spiral wrap 702 and cover sheath 704, such as at the proximal section 718, at the center section 716, and at the distal section 714. A source of welding energy, such as device 724, can provide an ultrasonic weld between the cover sheath 704 and the spiral wrap 702 by rotating the shaft 700, as indicated by the arrow 720, as device 724 emits ultrasonic energy to create circumferential welds 722 between the wraps of spiral wrap 702 and the inside of the cover sheath 704. In one embodiment, the welds 722 are a continuous band around the shaft 700. The welds 722 could be repeated at spaced intervals axially down the length of the shaft 700. The welds 722 restrict the amount of movement of the spiral wrap 702 allowed in relation to the cover sheath 704. An alternate embodiment in accordance with the present invention includes a method of applying a series of spot welds in a radial pattern.

Cover sheath 704 may be transparent to ultraviolet (UV) light. This allows a UV curable adhesive to be injected through the shaft to bond the spiral to the outer sheath in the required locations. This adhesive can then be cured by exposing it to UV light.

In designs where the spiral is made from a polyethylene, which is generally not suitable for adhesive bonding, adhesive can still be used to anchor the spiral to the outer sheath in the following manner. The adhesive can be injected through the sheath and will form ridges on the inner wall of the sheath that will protrude between the wraps of the spiral to anchor the spiral to the sheath.

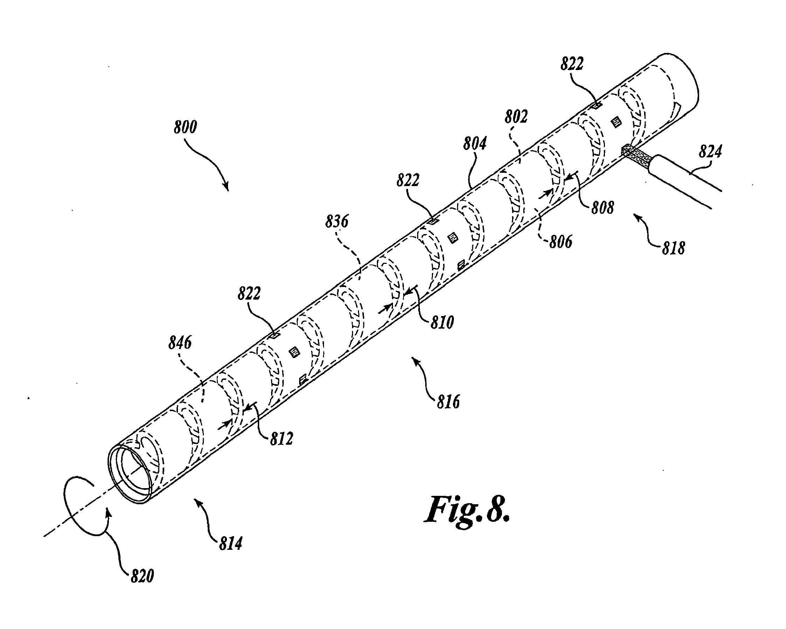

Referring to FIGURE 8, a shaft 800 having a spiral wrap 802 and cover sheath 804 is illustrated. The shaft 800 has a proximal section 818, a distal section 814, and a center section 816 between the proximal section 818 and the distal section 814. Wraps, such as wrap 806 in the proximal section 818, 836 in the center section 816, and wrap 846 in the distal section 814, may or may not have the same or different wrap width, wrap thickness, and gap spacing. The gap spacing 808 in the proximal section 818, or the gap spacing 810 in the center section 816, or the gap spacing 812 in the distal section 814 may or may not have been adjusted as described above. Through normal use of the shaft 800, however, the gap spacing 808 in the proximal section 818, center section 816, and distal section 814 can change throughout the life of the shaft 800. If the gap spacing increases, the increase results in greater flexibility (reduced stiffness) in the affected location. If the gap spacing decreases, the decrease results in reduced flexibility (increased stiffness) in the affected location. For a variety of reasons, it is desirable to maintain the gap spacing. To maintain the gap spacing at the desired setting, the cover sheath 804 is anchored to the spiral wrap 802 at more than one location along the axial direction of the shaft 800. In one embodiment, spot welds 822 are provided to weld wraps of the spiral wrap 802 to the cover sheath 804. Spot welds 822 may be provided at any location on the spiral wrap 802 and cover sheath 804, such as at the proximal section 818, at the center section 816, and at the distal section 814. A source of welding energy, such as device 824, can provide an ultrasonic spot weld between the cover sheath 804 and the spiral wrap 802 by rotating the shaft 800, as indicated by the arrow 820, as device 824 emits ultrasonic energy to create spot welds 822 between the wraps of spiral wrap 802 and the inside of the cover sheath 804. Spot welds 822 are made around one complete revolution of the shaft 800. The spot welds 822 could be repeated at intervals axially down the length of the shaft 800. The spot welds 822 restrict the amount of movement of the spiral wrap 802 allowed in relation to the cover sheath 804.

The materials used for the cover sheaths 704, 804 and spiral wraps 702, 802 are suitable to permit welds. Materials include polymers, such as polyethylene, high-density polyethylene, and polyurethane. If the outside diameter of the spiral wrap 702, 802, before assembly, is larger than the inside diameter of the cover sheath 704, 804 then the spiral wrap 702, 802 will press against the inside of the cover sheath 704, 804, when assembled. Welding requires contact between the parts to be welded.

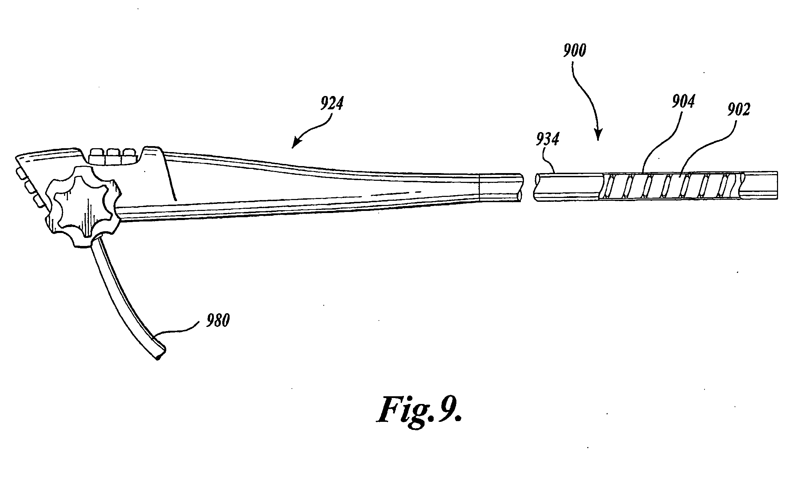

Referring to FIGURE 9, an endoscope 900 has a shaft 934 and a handle 924. The handle 924 is connected to a control unit (not shown) via the conduit 980. The shaft 934 is made from a spiral wrap 902 and a cover sheath 904. The spiral wrap 902 can be any one of the spirals made in accordance with an embodiment of the invention described herein. Although an endoscope 900 is illustrated, any medical device that tracks the anatomy can be provided with a shaft having a spiral wrap made in accordance with any one of the embodiments described above.

FIGURE 10 shows a slotted tube portion 1200 formed generally as a cylinder with an outer wall 1232 and a central lumen 1202. A series of slots 1220 are arranged around the outer circumference of the wall 1232 that allow the slotted tube to articulate. The wall thickness of the tube can be changed to increase or decrease the flexibility so that the slotted tube can replace any section of spiral wrap that is removed, as discussed above.

In another aspect, the shaft properties can be modified along the entire length, or by segments. For example, by selectively irradiating the desired portion of the shaft. This radiation can be of various spectra, for example, gamma particles, with the intent being to increase cross-linking of the shaft polymer material in the desired section.

While the preferred embodiment of the invention has been illustrated and described, it will be appreciated that various changes can be made therein without departing from the scope of the invention. It is therefore intended that the scope of the invention be determined from the following claims and equivalents thereof.

## Claims

1. A shaft (100, 300) for a medical device, comprising:
a cover sheath (104, 304);
a spiral wrap (102, 302) contained within the cover sheath (104, 304) and having a plurality of wraps (117, 119, 312, 314, 321) with gaps (106, 322, 324, 326) between the plurality of wraps (117, 119, 312, 314, 321), wherein the spiral wrap (102, 302) extends along the cover sheath (104, 304) from a proximal section (110, 312) to a center section (132, 314), and from the center section (132, 314) to a distal section (108, 321) of the spiral wrap (102, 302);
a change in the gap spacing (112, 114) between the plurality of wraps (117, 119) along the spiral wrap (102) between a first location (108), a second location (110), and a third location (132) of the cover sheath (104), wherein the proximal section (110) defines a first gap spacing (114), the distal section (108) defines a second gap spacing (112), and the center section (132) defines a third gap spacing that is greater than the first gap spacing (114) and less than the second gap spacing (112);
wherein the spiral wrap (102) is attached to the cover sheath (104), at least at the first, second and third locations (108, 110), thereby maintaining the change in gap spacing between the plurality of wraps (117, 119); and
wherein the proximal section (312) of the spiral wrap (302) defines a first inner diameter, the distal section (321) of the spiral wrap (302) defines a second inner diameter and the center section of the spiral wrap defines a third inner diameter, the second inner diameter being larger than the first and third inner diameters; and
wherein the spiral wrap (302) defines a substantially constant outer diameter (328).

2. The shaft of Claim 1, wherein the gap spacing (114) of the plurality of wraps (117) in the proximal section (110) is less than the gap spacing in a remainder of the spiral wrap (102).

3. The shaft of Claim 1, wherein the cover sheath (104, 304) is attached to the spiral wrap (102, 302) about 6 inches (152 mm) to about 12 inches (305 mm) measured from the end of the distal section (108, 310).

4. The shaft of Claim 1, wherein the cover sheath (104, 304) is attached to the spiral wrap (102, 302) about 8 inches (203 mm) to about 10 inches (254 mm) measured from the end of the distal section (108, 310).

5. The shaft of Claim 1, wherein the change in gap spacing (112, 114) provides about 1 inch (25.4 mm) of additional gap spacing in the distal section (108) as compared to a different section (110) of the spiral wrap (102).

6. The shaft of Claim 1, wherein the cover sheath (104, 304) is attached to the spiral wrap (102, 302) with one of adhesives, welding, or mechanical fasteners (118, 120).

7. The shaft of Claim 1, wherein at least one of the plurality of wraps (210) has a wrap width (222, 223) that is greater than the wrap width (224) of at least another of the plurality of wraps (216).

8. The shaft of Claim 1 wherein at least one of the plurality of wraps (102) has a wrap width from 3/8" (9.5 mm) to 1/2" (12.7 mm).

9. The shaft of Claim 1, wherein at least one of the plurality of wraps (312, 314) has a wrap thickness (318, 315) that is greater than a wrap thickness (320) of at least another of the plurality of wraps (321).

10. The shaft of Claim 1, wherein at least one of the plurality of wraps (134) is made of a material different than the material from which at least another of the plurality of wraps (134) is made.

11. The shaft of Claim 1 wherein the gap spacing (112) of the plurality of wraps (119) in the distal section (108) is greater than the gap spacing (114) in a remainder of the spiral wrap (102).

12. The shaft of Claim 1 wherein the distal section (321) has a wrap thickness (320) that is less than a wrap thickness (318) in the proximal section (312).

## Patentansprüche

1. Schaft (100, 300) für eine medizinische Vorrichtung, aufweisend:
eine Deckhülle (104, 304);
eine Spiral- bzw. Helixwicklung (102, 302), die innerhalb der Deckhülle (104, 304) angeordnet ist und mehrere Windungen (117, 119, 312, 314, 321) mit Zwischenräumen (106, 322, 324, 326) zwischen den mehreren Windungen (117, 119, 312, 314, 321) aufweist, wobei sich die Spiralwicklung (102, 302) entlang der Deckhülle (104, 304) von einem proximalen Abschnitt (110, 312) zu einem zentralen Abschnitt (132, 314) und von dem zentralen Abschnitt (132, 314) zu einem distalen Abschnitt (108, 321) der Spiralwicklung (102, 302) erstreckt;
eine Änderung der Zwischenraumweite (112, 114) zwischen den mehreren Windungen (117, 119) entlang der Spiralwicklung (102) zwischen einem ersten Ort (108), einem zweiten Ort (110) und einem dritten Ort (132) der Deckhülle (104), wobei der proximale Abschnitt (110) eine erste Zwischenraumweite (114) aufweist, der distale Abschnitt (108) eine zweite Zwischenraumweite (112) aufweist und der zentrale Abschnitt (132) eine dritte Zwischenraumweite aufweist, die größer ist als die erste Zwischenraumweite (114) und kleiner ist als die zweite Zwischenraumweite (112);
wobei die Spiralwicklung (102) zumindest an dem ersten, zweiten und dritten Ort (108, 110) an der Deckhülle (104) angebracht ist, um dadurch die Änderung der Zwischenraumweite zwischen den mehreren Windungen (117, 119) aufrechtzuerhalten; und
wobei der proximale Abschnitt (312) der Spiralwicklung (302) einen ersten Innendurchmesser aufweist, der distale Abschnitt (321) der Spiralwicklung (302) einen zweiten Innendurchmesser aufweist und der zentrale Abschnitt der Spiralwicklung einen dritten Innendurchmesser aufweist, wobei der zweite Innendurchmesser größer ist als der erste und der dritte Innendurchmesser; und
wobei die Spiralwicklung (302) einen im Wesentlichen konstanten Außendurchmesser (328) aufweist.

2. Schaft nach Anspruch 1, wobei die Zwischenraumweite (114) der mehreren Windungen (117) im proximalen Abschnitt (110) kleiner ist als die Zwischenraumweite im Rest der Spiralwicklung (102).

3. Schaft nach Anspruch 1, wobei die Deckhülle (104, 304) über etwa 6 Zoll (152mm) bis etwa 12 Zoll (305mm), gemessen vom Ende des distalen Abschnitts (108, 310), an der Spiralwicklung (102, 302) angebracht ist.

4. Schaft nach Anspruch 1, wobei die Deckhülle (104, 304) über etwa 8 Zoll (203mm) bis etwa 10 Zoll (254mm), gemessen vom Ende des distalen Abschnitts (108, 310), an der Spiralwicklung (102, 302) angebracht ist.

5. Schaft nach Anspruch l, wobei im distalen Abschnitt (108) die Änderung der Zwischenraumweite (112, 114) eine zusätzliche Zwischenraumweite von etwa 1 Zoll (25,4mm) bereitstellt, verglichen mit einem anderen Abschnitt (110) der Spiralwicklung.

6. Schaft nach Anspruch 1, wobei die Deckhülle (104, 304) mit Klebstoffen, durch Schweißen oder durch mechanische Befestigungsmittel (118, 120) an der Spiralwicklung (102, 302) angebracht ist.

7. Schaft nach Anspruch 1, wobei mindestens eine der mehreren Windungen (210) eine Windungsbreite (222, 223) hat, die größer ist als die Windungsbreite (224) mindestens einer anderen der mehreren Windungen (216).

8. Schaft nach Anspruch 1, wobei mindestens eine der mehreren Windungen (102) eine Windungsbreite von 3/8 Zoll (9,5mm) bis 1/2 Zoll (12,7mm) hat.

9. Schaft nach Anspruch 1, wobei mindestens eine der mehreren Windungen (312, 314) eine Windungsdicke (318, 315) hat, die größer ist als eine Windungsdicke (320) mindestens einer anderen der mehreren Windungen (321).

10. Schaft nach Anspruch 1, wobei mindestens eine der mehreren Windungen (134) aus einem Material hergestellt ist, das sich von dem Material unterscheidet, aus dem mindestens eine andere der mehreren Windungen (134) hergestellt ist.

11. Schaft nach Anspruch 1, wobei die Zwischenraumweite (112) der mehreren Windungen (119) im distalen Abschnitt (108) größer ist als die Zwischenraumweite (114) im Rest der Spiralwicklung (102).

12. Schaft nach Anspruch 1, wobei der distale Abschnitt (321) eine Windungsdicke (320) hat, die kleiner ist als die Windungsdicke (318) im proximalen Abschnitt (312).

## Revendications

1. Tige (100, 300) pour un dispositif médical, présentant :
une gaine de couverture (104, 304) ;
un enroulement Spiral et/ou hélicoïdal (102, 302) contenu à l'intérieur de la gaine de couverture (104, 304) et présentant une pluralité de spires (117, 119, 312, 314, 321) avec des interstices (106, 322, 324, 326) entre la pluralité de spires (117, 119, 312, 314, 321), l'enroulement spiral (102, 302) s'étendant le long de la gaine de couverture (104, 304) d'une section proximale (110, 312) à une section centrale (132, 314), et de la section centrale (132, 314) à une section distale (108, 321) de l'enroulement spiral (102, 302) ;
une variation de la largeur d'interstice (112, 114) entre la pluralité de spires (117, 119) le long de l'enroulement spiral (102) entre un premier emplacement (108), un deuxième emplacement (110) et un troisième emplacement (132) de la gaine de couverture (104),
la section proximale (110) définissant une première largeur d'interstice (114), la section distale (108) définissant une deuxième largeur d'interstice (112), et la section centrale (132) définissant une troisième largeur d'interstice supérieure à la première largeur d'interstice (114) et inférieure à la deuxième largeur d'interstice (112) ;
l'enroulement spiral (102) étant fixé à la gaine de couverture (104), au moins sur le premier, le deuxième et le troisième emplacements (108, 110), en maintenant ainsi la variation de largeur d'interstice entre la pluralité de spires (117, 119) ; et
la section proximale (312) de l'enroulement spiral (302) définissant un premier diamètre intérieur, la section distale (321) de l'enroulement spiral (302) définissant un deuxième diamètre intérieur, et la section centrale de l'enroulement spiral définissant un troisième diamètre intérieur, le deuxième diamètre intérieur étant supérieur au premier et au troisième diamètres intérieurs ; et
l'enroulement spiral (302) définissant un diamètre extérieur (328) sensiblement constant.

2. Tige selon la revendication 1, où la largeur d'interstice (114) de la pluralité de spires (117) est inférieure dans la section proximale (110) à la largeur d'interstice dans le reste de l'enroulement spiral (102).

3. Tige selon la revendication 1, où la gaine de couverture (104, 304) est fixée à l'enroulement spiral (102, 302) sur une longueur comprise entre 6 pouces (152 mm) environ et 12 pouces (305 mm) environ, mesurés depuis l'extrémité de la section distale (108, 310).

4. Tige selon la revendication 1, où la gaine de couverture (104, 304) est fixée à l'enroulement spiral (102, 302) sur une longueur comprise entre 8 pouces (203 mm) environ et 10 pouces (254 mm) environ, mesurés depuis l'extrémité de la section distale (108,310).

5. Tige selon la revendication 1, où la variation de la largeur d'interstice (112, 114) ajoute 1 pouce (25,4 mm) de largeur d'interstice dans la section distale (108) par rapport à une autre section (110) de l'enroulement spiral (102).

6. Tige selon la revendication 1, où la gaine de couverture (104, 304) est fixée à l'enroulement spiral (102, 302) par un des moyens suivants : adhésif, soudage ou fixations mécaniques (118, 120).

7. Tige selon la revendication 1, où au moins une spire de la pluralité de spires (210) a une largeur de spire (222, 223) supérieure à la largeur de spire (224) d'au moins une autre spire de la pluralité de spires (216).

8. Tige selon la revendication 1, où au moins une spire de la pluralité de spires (102) a une largeur de spire comprise entre 3/8e de pouce (9,5 mm) et 1/2 pouce (12,7 mm).

9. Tige selon la revendication 1, où au moins une spire de la pluralité de spires (312, 314) a une épaisseur de spire (318, 315) supérieure à l'épaisseur de spire (320) d'au moins une autre spire de la pluralité de spires (321).

10. Tige selon la revendication 1, où au moins une spire de la pluralité de spires (134) est constituée d'un matériau différent du matériau dont est constituée au moins une autre spire de la pluralité de spires (134).

11. Tige selon la revendication 1, où la largeur d'interstice (112) de la pluralité de spires (119) dans la section distale (108) est supérieure à la largeur d'interstice (114) du reste l'enroulement spiral (102).

12. Tige selon la revendication 1, où la section distale (321) présente une épaisseur de spire (320) inférieure à l'épaisseur de spire (318) dans la section proximale (312).
